# EUROPEAN PATENT SPECIFICATION

(11) **EP 3 595 813 B1**
(45) Date of publication and mention of the grant of the patent: **14.04.2021**
(21) Application number: 18712442.5
(22) Date of filing: 12.03.2018
(51) Int. Cl.: B01L 3/00, C12M 3/04, C12M 1/00, G01N 23/00

(54) **A SYSTEM AND A METHOD FOR IRRADIATING BIOLOGICAL MATERIAL**
SYSTEM UND VERFAHREN ZUR BESTRAHLUNG VON BIOLOGISCHEM MATERIAL
SYSTÈME ET PROCÉDÉ D'EXPOSITION D'UNE MATIÈRE BIOLOGIQUE À UN RAYONNEMENT

(30) Priority: 14.03.2017 FI 20175229
(43) Date of publication of application: 22.01.2020
(73) Proprietor: Biogenium Microsystems Oy, 33720 Tampere (FI)
(72) Inventor: KALLIO, Pasi, 33820 Tampere (FI); RANTANEN, Krista, 24100 Salo (FI); KREUTZER, Joose, 33710 Tampere (FI); TANHUANPÄÄ, Olli, 33720 Tampere (FI); JAAKKOLA, Panu, 20540 Turku (FI)
(74) Representative: Finnpatent Oy
(86) International application number: PCT/FI2018/050176
(87) International publication number: WO 2018/167360

(56) References cited:
- CN-U- 203 639 467
- W. TINGANELLI ET AL: "Influence of acute hypoxia and radiation quality on cell survival", JOURNAL OF RADIATION RESEARCH, vol. 54, no. suppl 1, 1 July 2013 (2013-07-01), pages i23-i30, XP055469411, JP ISSN: 0449-3060, DOI: 10.1093/jrr/rrt065
- KREUTZER JOOSE ET AL: "Cell culture chamber with gas supply for prolonged recording of human neuronal cells on microelectrode array", JOURNAL OF NEUROSCIENCE METHODS, vol. 280, 1 February 2017 (2017-02-01), pages 27-35, XP029939899, ISSN: 0165-0270, DOI: 10.1016/J.JNEUMETH.2017.01.019
- MARTIN D. BRENNAN ET AL: "A 3D-Printed Oxygen Control Insert for a 24-Well Plate", PLOS ONE, vol. 10, no. 9, 11 September 2015 (2015-09-11), page e0137631, XP055469223, DOI: 10.1371/journal.pone.0137631
- Joose Kreutzer ET AL: "Mini-incubator For Prolonged Cell Culture And Hypoxia Studies Outside An Incubator", MEA Meeting 2016 | 10th International Meeting on Substrate-Integr ated Electrode Arrays,, 24 June 2016 (2016-06-24), XP055469049, Reutlingen, Germany Retrieved from the Internet: URL:https://www.frontiersin.org/10.3389/co nf.fnins.2016.93.00043/event_abstract [retrieved on 2018-04-20]

## Description

### Field of the disclosure

The disclosure relates to a system and to a method for irradiating biological material in a controlled gas environment. The biological material can be, for example but not necessarily, a cell culture or a sample of tissue extracted from a living organism.

### Background

In conjunction with many research projects there can be a need to irradiate a cell culture or other biological material in controlled gas environments. For example, there can be a need to irradiate a cell culture with electromagnetic radiation such as e.g. X-rays or ultraviolet rays and/or with particle radiation such as e.g. α- or β-radiation so that the cells are under hypoxic conditions during the irradiation process. Hypoxia research is an example of such research areas that suffer of environmental changes directed to cell cultures. Therefore, studying the effect of radiation on living cells which are under hypoxic conditions requires continuous control and management of the gas environment of the cells being irradiated.

Commercial hypoxia chambers are available but they are very expensive to purchase and maintain, and thus they are not available for all research groups. Moreover, commercial hypoxia chambers are large hoods which do not provide fast dynamic changes in the gas environment of a cell culture. It might take several hours to stabilize the gas environment after a change. Furthermore, it may be challenging to arrange a radiation source to operate inside a hypoxia chamber of the kind mentioned above so that a desired dose of radiation is directed to a cell culture. Thus, there can be a need to move the cell culture out from the hypoxia chamber in order to irradiate the cells. As a corollary, low oxygen concentration disappears rapidly from the gas environment of the cell culture. This might create severe consequences to the cells. Therefore, in this exemplifying research area it is important to keep the cell culture in the same conditions throughout the culture process and also during the irradiation process.

Publication W. Tinganelli et al.: "Influence of acute hypoxia and radiation quality on cell survival", Journal of Radiator Research, vol. 54, no. suppl 1, 1 July 2013 presents experiments that have been performed using Chinese hamster ovary "CHO" cells and RAT-1 rat prostate cancer cells to find out effect of acute oxygen depletion on cell survival for different types of radiation.

Publication CN203639467 describes a small portable cell hypoxia training system that comprises a hypoxia environment cell culture tank, an oxygen cylinder, and a nitrogen cylinder. The oxygen cylinder and the nitrogen cylinder are respectively connected with different air inlets of the culture tank through a decrement gauge and an electromagnetic valve. An oxygen sensor is arranged inside the culture tank, and a controller unit is arranged on the system and connected with the oxygen sensor and the electromagnetic valve. Oxygen concentration value inside the culture tank is measured by the oxygen sensor, and the electromagnetic valves are controlled according to the oxygen concentration value.

Publication Kreutzer Joose et al.: "Cell culture chamber with gas supply for prolonged recording of human neuronal cells on microelectrode array", Journal of Neuroscience Methods, vol. 280, 1 February 2017, pages 27-35 demonstrates a cell culture chamber that enables stable culture conditions during prolonged extracellular recordings on a microelectrode array "MEA" outside an incubator. The cell culture chamber consists of a gas permeable silicone structure that enables gas transfer into the chamber. The obtained results show that the culture chamber supports the growth of the human embryonic stem cell "hESC" derived neurons both inside and outside an incubator. The structure provides very low evaporation, stable pH and osmolarity, and maintains strong signaling of hESC-derived neuronal networks over three-day MEA experiments.

Publication Martin D. Brennan et al.: "A 30-Printed Oxygen Control Insert for a 24-Well Plate", Plos One, vol. 10, no. 9, 11 September 2015 demonstrates an addition of gas permeable PDMS "Polydimethylsiloxane" membranes to 3D-printed microfluidic devices as means to enable oxygen control cell culture studies. The incorporation of a 3D-printed device and gas-permeable membranes is demonstrated on a 24-well oxygen control device for standard multiwell plates. The direct printing allows integrated distribution channels and device geometries not possible with traditional planar lithography.

### Summary

The following presents a simplified summary in order to provide a basic understanding of some aspects of various invention embodiments. The summary is not an extensive overview of the invention. It is neither intended to identify key or critical elements of the invention nor to delineate the scope of the invention. The following summary merely presents some concepts of the invention in a simplified form as a prelude to a more detailed description of exemplifying and non-limiting embodiments of the invention.

In accordance with the invention, there is provided a new system for irradiating biological material such as for example a cell culture or a sample of tissue extracted from a living organism. The system comprises:
- a platform for holding the biological material, and
- a radiation source for directing at least one of the following radiations to the biological material: X-ray radiation, ultraviolet radiation, particle radiation.

The platform comprises one or more platform elements each comprising a chamber for containing the biological material. The platform further comprises a frame structure for mechanically supporting the platform elements and for mechanically supporting a gas-supply system capable of supplying gas to the platform elements without receiving the gas from an external source. The frame structure comprises gas channels for receiving the gas from the gas-supply system and for conducting the gas to the platform elements so as to determine gas composition in each of the chambers during irradiation of the biological material. Each of the platform elements comprises:
- a first reservoir for containing liquid-form culturing medium,
- a first liquid duct for conducting the liquid-form culturing medium from the first reservoir to the chamber of the platform element under consideration, and
- a second liquid duct for conducting the liquid-form culturing medium out from the chamber of the platform element under consideration.

The above-mentioned radiation source can be, for example but not necessarily, a cesium Cs-137 radiation source, an Iridium Ir-192 radiation source, a iodine 1-125 radiation source, a cobalt Co-60 radiation source, or a radium Ra-226 radiation source

The above-described platform and the gas-supply system are capable of constituting a portable device which maintains a desired gas environment of the biological material. Thus, moving the portable device to a place where the irradiation process can be carried out does not cause a change in the gas environment of the biological material. Therefore, the above-described system makes it possible to examine for example how radiation effects on a cell culture under hypoxia conditions.

In accordance with the invention, there is provided also a new method for irradiating biological material such as for example a cell culture or a sample of tissue extracted from a living organism. The method comprises:
- holding the biological material in one or more chambers of one or more platform elements of a system according to the invention,
- maintaining, in the one or more chambers containing the biological material, a desired gas composition different from that of the ambient air, and
- activating the radiation source of the system to direct at least one of the following radiations to the biological material: X-ray radiation, ultraviolet radiation, particle radiation.

A number of exemplifying and non-limiting embodiments of the invention are described in accompanied dependent claims.

Various exemplifying and non-limiting embodiments of the invention both as to constructions and to methods of operation, together with additional objects and advantages thereof, will be best understood from the following description of specific exemplifying embodiments when read in connection with the accompanying drawings.

The verbs "to comprise" and "to include" are used in this document as open limitations that neither exclude nor require the existence of also un-recited features. The features recited in dependent claims are mutually freely combinable unless otherwise explicitly stated. Furthermore, it is to be understood that the use of "a" or "an", i.e. a singular form, throughout this document does not exclude a plurality.

### Brief description of figures

Exemplifying and non-limiting embodiments of the invention and their advantages are explained in greater detail below with reference to the accompanying drawings, in which:
figure 1a illustrates the mechanical structure of a system according to an exemplifying and non-limiting embodiment of the invention for irradiating biological material,
figure 1b illustrates the operation of a system according to an exemplifying and non-limiting embodiment of the invention for irradiating biological material,
figures 2a-2g illustrate the mechanical structure of platform elements of system illustrated in figure 1a, and
figure 3 shows a flowchart of a method according to an exemplifying and non-limiting embodiment of the invention for irradiating biological material.

### Description of exemplifying and non-limiting embodiments

The specific examples provided in the description given below should not be construed as limiting the scope and/or the applicability of the appended claims. Lists and groups of examples provided in the description given below are not exhaustive unless otherwise explicitly stated.

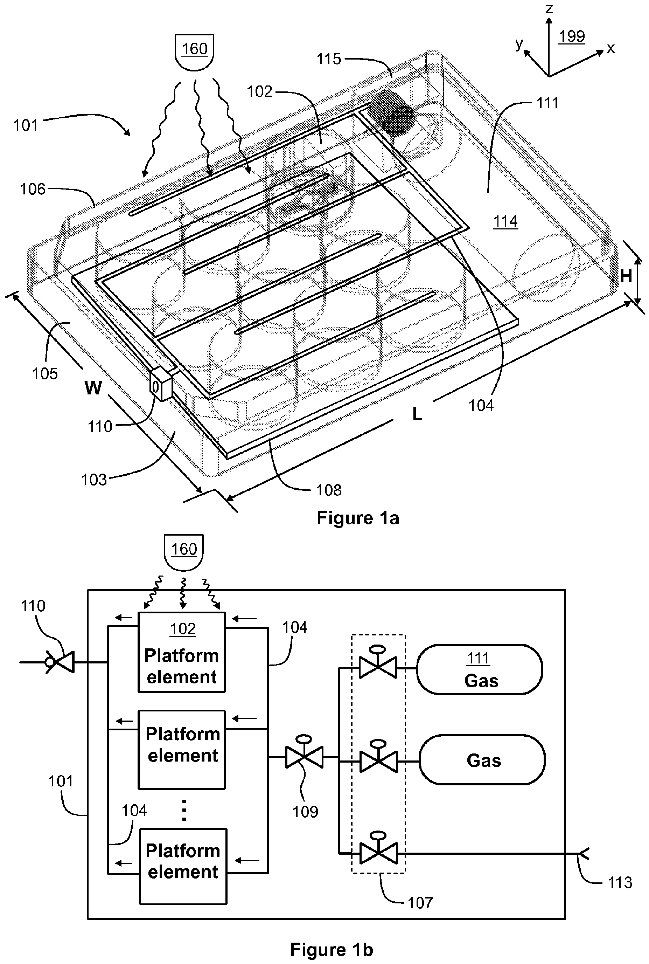

Figure 1a shows the mechanical structure of a system according to an exemplifying and non-limiting embodiment of the invention for irradiating biological material. The system comprises a platform 101 and a gas-supply system 111 that is capable of supplying gas having a desired composition without receiving the gas from an external source. The system comprises a radiation source 160 for directing X-ray radiation to the biological material. In addition to or instead of X-rays, the radiation source can emit e.g. visible light, ultraviolet "UV" radiation, or some other electromagnetic radiation, and/or particle radiation such as e.g. α- or β-radiation. The platform 101 comprises platform elements each of which comprises a chamber for containing the biological material. In figure 1a, one of the platform elements is denoted with a figure reference 102. The other platform elements are not shown in figure 1a. The platform 101 further comprises a frame structure 103 for mechanically supporting the platform elements and the gas-supply system 111. The frame structure 103 comprises gas channels 104 for receiving the gas from the gas-supply system 111 and for conducting the gas to the platform elements. In this exemplifying case, the platform elements, such as the platform element 102, are separate components with respect to the frame structure 103. It is however also possible that each platform element is an integral part of the frame structure. The platform elements can be made of for example polydimethylsiloxane "PDMS".

In the exemplifying system illustrated in figure 1a, the platform 101 is suitable for acting as a cell culture platform and each of the platform elements is suitable for acting as a culture element where the chamber for containing biological material is suitable for acting as a culture chamber for containing cultured cells and liquid form culturing medium. Each of the platform elements may comprise a gas room for containing the above-mentioned gas and gas permeable material between the gas room and the chamber for containing biological material. The gas channels 104 are advantageously arranged to conduct the gas to flow through the platform elements so that the gas in the platform elements can be changed rapidly if needed.

The frame structure 103 is advantageously at least partly made of one or more transparent materials. Correspondingly, each platform element is advantageously at least partly made of one or more transparent materials. In this exemplifying case, the biological material can be imaged and/or examined using optical microscopy techniques. The one or more transparent materials may comprise for example polystyrene, polystyrene with copolymers, polyvinyl chloride, polyvinyl chloride with copolymers, polyethylene, polystyrene-acrylonitrile, polypropylene, polyvinylidine chloride, polycarbonate, cyclic olefin copolymer, and/or silicone elastomer.

In the exemplifying platform 101 illustrated in figure 1a, the frame structure 103 comprises a well plate 105 and a lid 106. The well plate 105 comprises wells each being capable of containing one of the platform elements, and the lid 106 comprises the above-mentioned gas channels 104. The lid 106 closes the wells when the lid is placed on top of the well plate as illustrated in figure 1a. The physical dimensions of the well plate 105 are advantageously similar to the physical dimensions of a standard laboratory well plate so that the platform 101 can be easily placed on e.g. a sample table of a microscope and/or on another instrument. The length L of the platform 101 can be e.g. on the range from 86 mm to 128 mm, the width W of the platform 101 can be e.g. on the range from 60 mm to 86 mm, and the height H of the platform 101 can be e.g. on the range from 18 mm to 45 mm.

In the exemplifying system illustrated in figure 1a, the gas-supply system 111 comprises a replaceable container 114 that contains pressurized gas. The replaceable container 114 can be for example a gas cartridge. The replaceable container 114 may contain for example 12 g gas the volume of which is about 7 liters in the normal temperature and pressure "NTP". With a suitable design of the platform 101, the above-mentioned volume can be enough to maintain sufficient gas flows through individual platform elements for several days. The platform elements are advantageously designed so that a small gas flow rate, e.g. about 100 µl/min, is enough to maintain the desired gas environment inside the platform elements. Using the replaceable container 114 it is not necessary to use a gas pipe connected to an external gas supply system. Therefore, the platform 101 and the gas-supply system 111 constitute a portable device where the gas environment of biological material can be maintained because the gas-supply system 111 is capable of operating autonomously without connections to an external gas supply system. Moving the portable device to a place where an irradiation process can be carried out does not cause a change in the gas environment of the biological material. Therefore, it is possible to examine, for example, how X-ray radiation effects on a cell culture under hypoxia conditions. Furthermore, for example microscopic imaging that may be needed for monitoring the biological material does not cause a change in the gas environment of the biological material. In the exemplifying case illustrated in figure 1a, the frame structure 103 comprises a cavity for the replaceable container 114. It is, however, also possible that the frame structure 103 comprises means for fastening a container for pressurized gas to an outer surface of the frame structure.

It is worth noting that the above-mentioned replaceable container 114 is not the only possible choice for the gas-supply system 111. It is also possible that the gas-supply system 111 comprises a refillable container for pressurized gas. The refillable container comprises a refilling valve for enabling the refilling from an external source such as a gas bottle and/or a gas mixer. Furthermore, it is also possible that the gas-supply system comprises a room for containing liquids which interact so that desired gas is generated.

The gas flow in a system according to an exemplifying and non-limiting embodiment of the invention is illustrated in figure 1b. The platform 101 of the system comprises the gas channels 104 for receiving the gas from the gas-supply system 111 and for conducting the gas to flow through the platform elements one of which is denoted with the figure reference 102. The platform 101 may comprise a controllable valve 109 for controlling the gas flow through the platform elements. In the exemplifying system illustrated in figure 1a, the controllable valve can be located in an element 115. The platform 101 may further comprise a backflow barrier for preventing the ambient air from flowing into the platform elements in a direction opposite to the flowing direction of the gas supplied by the gas-supply system. In figures 1a and 1b, the backflow barrier is denoted with a figure reference 110.

In a system according to an exemplifying and non-limiting embodiment of the invention, the frame structure 103 is configured to mechanically support two or more autonomous gas-supply systems. In this exemplifying case, the gas channels may comprise a selection valve system for selecting which one of the gas-supply systems is enabled to supply gas to the platform elements. In figure 1b, the selection valve system is denoted with a figure reference 107. With for example two replaceable containers, e.g. gas cartridges, carried by the frame structure it is possible to maintain a desired gas environment of the biological material during a change of a replaceable container. Furthermore, one of the gas-supply systems may contain different gas than another of the gas-supply systems. In this exemplifying case, it is possible to alter the gas environment of the biological material in a controlled way. For example, in conjunction with hypoxia research, it is possible to perform reoxygenation experiments so that two different gas concentrations are supplied in relays.

In a system according to an exemplifying and non-limiting embodiment of the invention, the gas channels comprise a pipe interface 113 for connecting to an external gas supply system. In this exemplifying case, an external gas bottle and/or a gas mixer can be connected to the platform 101 with a gas pipe in order to avoid consuming the gas from a replaceable and/or refillable container, e.g. a gas cartridge. In the exemplifying system illustrated in figure 1a, a pipe interface for connecting to an external gas supply system can be located in the element 115.

A system according to an exemplifying and non-limiting embodiment of the invention comprises an electrically operated heating element for controlling temperature of biological material contained by the above-mentioned platform elements. In the exemplifying system illustrated in figure 1a, the heating element is denoted with a figure reference 108. The heating element 108 may comprise for example resistor wires which are advantageously so thin that they do not disturb for example microscope imaging and other optical operations. The heating element 108 can be for example battery operated, and the frame structure 103 can be arranged to mechanically support the battery.

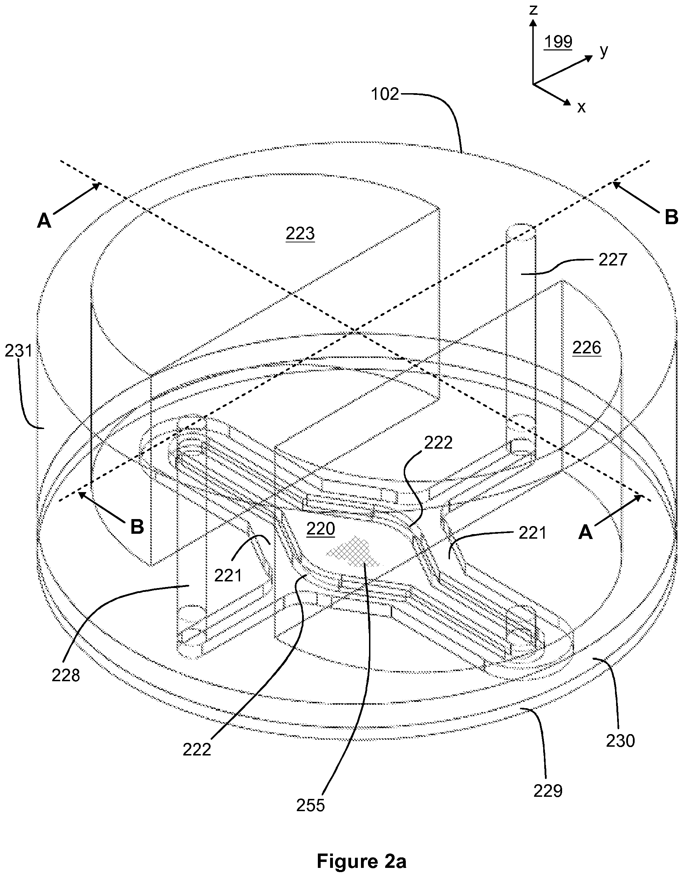

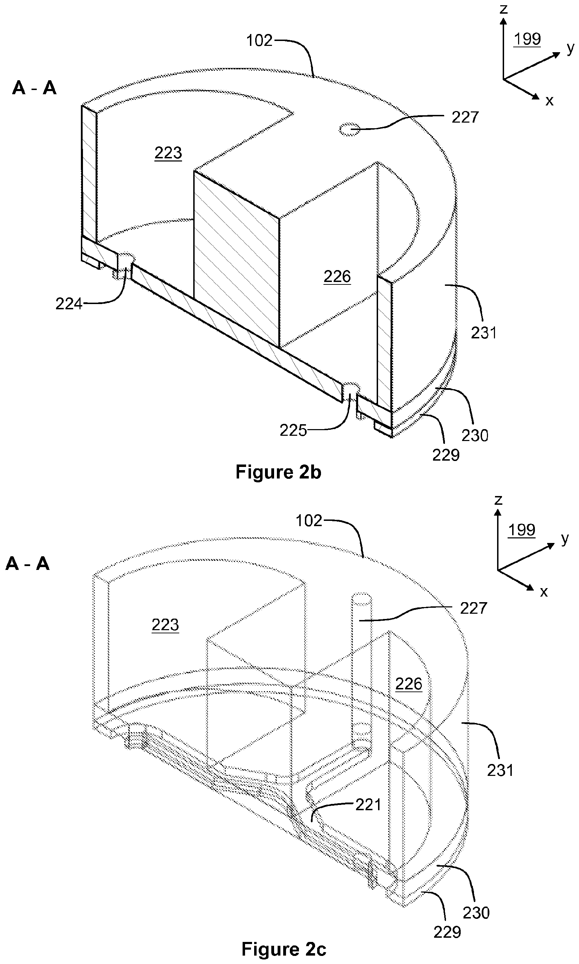

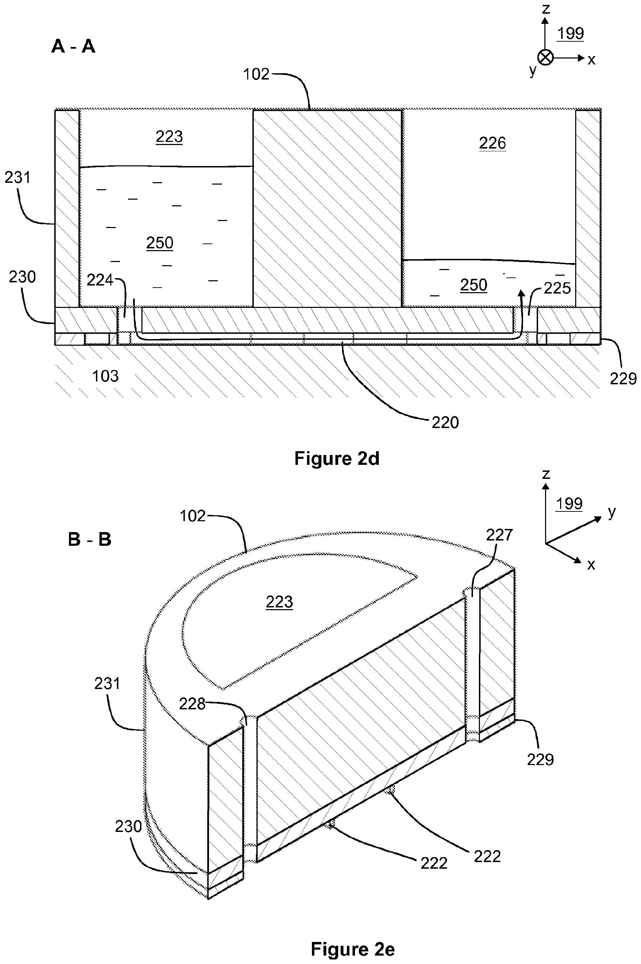

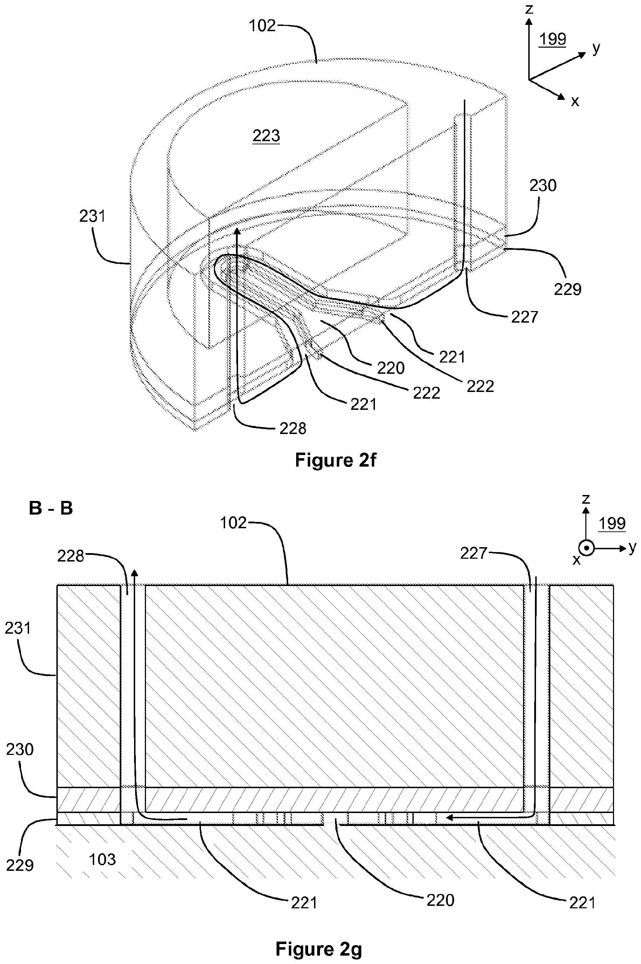

Figure 2a illustrates the platform element 102 shown in figure 1a. Figures 2b, 2c and 2d show a section obtained by cutting the platform element 102 along a line A-A shown in figure 2a. The section plane related to figures 2b-2d is parallel with the xz-plane of a coordinate system 199. In figures 2b and 2d, the section surfaces are denoted with diagonal hatchings. Figures 2e, 2f, and 2g show a section obtained by cutting the platform element 102 along a line B-B shown in figure 2a. The section plane related to figures 2e-2g is parallel with the yz-plane of the coordinate system 199. The viewing directions related to figures 1a and 2a-2g are illustrated by the coordinate system 199.

The platform element 102 comprises a chamber that is suitable for containing e.g. a cell culture and liquid-form culturing medium. The chamber is denoted with a figure reference 220 in figures 2a, 2d, 2f, and 2g. In figure 2a, the cell culture is depicted with a cross-hatched area 255. The platform element 102 comprises a gas room for containing the gas which is used for providing the desired gas environment for the cell culture. The gas room is denoted with a figure reference 221 in figures 2a, 2c, 2f, and 2g. The platform element 102 comprises gas permeable material between the gas room 221 and the chamber 220. The gas permeable material is denoted with a figure reference 222 in figures 2a, 2e, and 2f. The gas permeable material may comprise for example polydimethylsiloxane "PDMS".

The gas room 221 and the chamber 220 are advantageously separated from each other with water impermeable structures which prevent water from leaking from the chamber to the gas room. An advantage achieved with the water impermeable structures is that the gas can be kept dry, and dry gas makes it possible to use lower gas flow rates and thereby to save gas. Furthermore, the water tight chamber is capable of effectively protecting a cell culture against contaminations.

The exemplifying platform element 102 further comprises a first reservoir for containing the liquid-form culturing medium, a first liquid duct for conducting the liquid-form culturing medium from the first reservoir to the chamber 220, a second liquid duct for conducting the liquid-form culturing medium out from the chamber 220, and a second reservoir 226 connected to the second liquid duct. The above-mentioned first reservoir is denoted with a figure reference 223 in figures 2a-2f, the above-mentioned first liquid duct is denoted with a figure reference 224 in figures 2b and 2d, the above-mentioned second liquid duct is denoted with a figure reference 225 in figures 2b and 2d, and the above-mentioned second reservoir is denoted with a figure reference 226 in figures 2a-2d. In figure 2d, the liquid-form culturing medium is denoted with a figure reference 250 and the flow of the liquid-form culturing medium from the first reservoir 223 through the culture chamber 220 to the second reservoir 226 is depicted with a curved line arrow. In the exemplifying situation shown in figure 2d, the flow is based on a hydrostatic pressure difference between the first and second reservoirs 223 and 226. As can be seen from figure 2d, the frame structure 103 constitutes the bottom for the platform element 102. It is, however, also possible that the platform element 102 has its own bottom element.

The exemplifying platform element 102 further comprises a first gas duct for conducting the gas from the gas channels 104 shown in figures 1a and 1b to the gas room 221 and a second gas duct for conducting the gas out from the gas room 221. The first gas duct is denoted with a figure reference 227 in figures 2a, 2b, 2c, 2e, 2f and 2g, and the second gas duct is denoted with a figure reference 228 in figures 2a, 2e, 2f, and 2g. In figures 2f and 2g, the gas flow is depicted with curved line arrows.

As illustrated in figure 2a, the gas room 221 of the platform element 102 surrounds the chamber 220, and a wall between the gas room and the culture chamber comprises the gas permeable material 222. As shown in figure 2a, the gas room has an elongated shape so that the gas room is actually a gas channel and thus the volume of the gas room 221 can be relatively small. The small volume of the gas room facilitates making fast changes, if needed, in the gas environment of the cell culture or other biological material. For example, the volume of the gas room 221 can be from about 0.5 to about 3 times the volume of the chamber 220 for containing the cell culture or other biological material.

The mechanical structure of the exemplifying platform element 102 illustrated in figures 2a-2g comprises portions stacked on top of each other. In figures 2a-2g, the above-mentioned portions are denoted with figure references 229, 230, and 231. As shown in figures 2a-2g, the portions 229-231 are stacked on each other in the z-direction of the coordinate system 199. As can be seen from figures 2a-2g, each of the portions 229-231 has a constant cross-sectional profile when a section plane is perpendicular to the z-axis of the coordinate system 199.

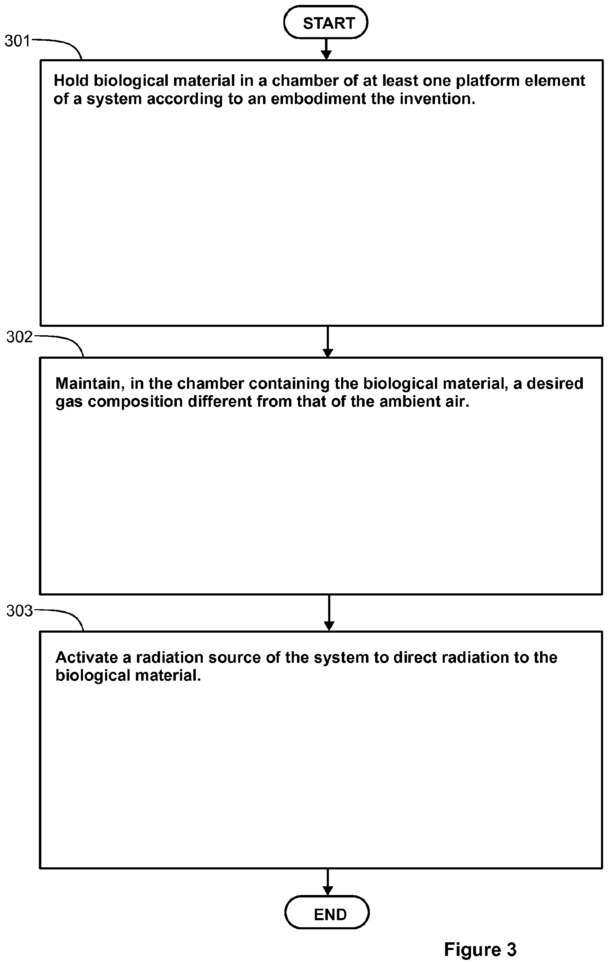

Figure 3 shows a flowchart of a method according to an exemplifying and non-limiting embodiment of the invention for irradiating biological material such as for example a cell culture or a sample of tissue extracted from a living organism. The method comprises:
- action 301: holding the biological material in one or more chambers of one or more platform elements of a system according to an embodiment of the invention,
- action 302: maintaining, in the one or more chambers containing the biological material, a gas composition different from that of the ambient air, and
- action 303: activating the radiation source of the system to direct X-ray radiation to the biological material.

In a method according to an exemplifying and non-limiting embodiment of the invention, the gas contains less oxygen than the ambient air so as to create hypoxia conditions for the biological material.

In a method according to an exemplifying and non-limiting embodiment of the invention, the system comprises an electrically operated heating element and the temperature of the biological material is controlled with the aid of the electrically operated heating element.

In a method according to an exemplifying and non-limiting embodiment of the invention, the platform elements and a frame structure mechanically supporting the platform elements are at least partly made of one or more transparent materials, and the biological material is optically imaged and/or otherwise inspected with the aid of a microscope.

In a method according to an exemplifying and non-limiting embodiment of the invention, the biological material comprises one or more cell cultures cultured in the above-mentioned system.

The non-limiting, specific examples provided in the description given above should not be construed as limiting the scope and/or the applicability of the appended claims. Lists and groups of examples provided in the description given above are not exhaustive unless otherwise explicitly stated.

## Claims

1. A system for irradiating biological material, the system comprising:
- a platform (101) for holding the biological material, and
- a radiation source (160) for directing at least one of the following radiations to the biological material: X-ray radiation, ultraviolet radiation, particle radiation,
wherein the platform comprises one or more platform elements (102) each comprising a chamber (220) for containing the biological material, wherein the platform further comprises a frame structure (103) for mechanically supporting the platform elements and for mechanically supporting a gas-supply system capable of supplying gas to the platform elements without receiving the gas from an external source, and the frame structure comprises gas channels (104) for receiving the gas from the gas-supply system and for conducting the gas to the platform elements so as to determine gas composition in each of the chambers during irradiation of the biological material, **characterized in that** each of the platform elements comprises:
- a first reservoir (223) for containing liquid-form culturing medium,
- a first liquid duct (224) for conducting the liquid-form culturing medium from the first reservoir to the chamber of the platform element under consideration, and
- a second liquid duct (225) for conducting the liquid-form culturing medium out from the chamber of the platform element under consideration.

2. A system according to claim 1, wherein each of the platform elements comprises a gas room (221) for containing the gas and gas permeable material (222) between the gas room and the chamber.

3. A system according to claim 2, wherein the gas room (221) of each of the platform elements surrounds the chamber (220) of the platform element under consideration, and a wall between the gas room and the chamber comprises the gas permeable material.

4. A system according to claim 2 or 3, wherein each of the platform elements comprises:
- a first gas duct (227) for conducting the gas from the gas channels to the gas room (221) of the platform element under consideration, and
- a second gas duct (228) for conducting the gas out from the gas room of the platform element under consideration.

5. A system according to any of claims 2-4, wherein a volume of the gas room of each of the platform elements is from 0.5 to 3 times, preferably from 0.5 to 1 times, a volume of the chamber of the platform element under consideration.

6. A system according to any of claims 2-5, wherein the gas permeable material (222) comprises polydimethylsiloxane.

7. A system according to any of claims 2-6, wherein the gas room (221) and the chamber (220) are separated from each other with water impermeable structures for preventing water from leaking from the chamber to the gas room.

8. A system according to any of claims 1-7, wherein the gas channels (104) are arranged to conduct the gas to flow through the platform elements.

9. A system according to claim 8, wherein the platform further comprises a backflow barrier (110) for preventing ambient air from flowing into the platform elements in a direction opposite to a flowing direction of the gas.

10. A system according to any of claims 1-9, wherein the platform is a cell culture platform and each of the platform elements is a culture element in which the chamber for containing the biological material is a culture chamber for containing a cell culture and liquid form culturing medium.

11. A system according to any of claims 1-10, wherein the frame structure comprises a well plate (105) comprising wells each being capable of containing one of the platform elements, and a lid (106) comprising the gas channels and for closing the wells when being placed on top of the well plate.

12. A system according to claim 11, wherein the well plate (105) comprises a cavity for containing the gas-supply system.

13. A system according to any of claims 1-12, wherein the frame structure is configured to mechanically support another gas-supply system, and the gas channels comprise a selection valve system (107) for selecting which one of the gas-supply systems is enabled to supply the gas to the platform elements.

14. A system according to claim 1, wherein each of the platform elements comprises a second reservoir (226) connected to the second liquid duct of the platform element under consideration.

15. A method for irradiating biological material, **characterized in that** the method comprises:
- holding (301) the biological material in one or more chambers of one or more platform elements of a system according to any of claims 1-14,
- maintaining (302), in the one or more chambers containing the biological material, a gas composition different from that of ambient air, and
- activating (303) the radiation source of the system to direct at least one of the following radiations to the biological material: X-ray radiation, ultraviolet radiation, particle radiation.

## Patentansprüche

1. System zur Bestrahlung von biologischem Material, wobei das System Folgendes umfasst:
- eine Plattform (101) zum Halten des biologischen Materials und
- eine Strahlungsquelle (160) zum Lenken mindestens einer der folgenden Strahlungen auf das biologische Material: Röntgenstrahlung, ultraviolette Strahlung, Teilchenstrahlung,
wobei die Plattform ein oder mehrere Plattformelemente (102) umfasst, die jeweils eine Kammer (220) zum Aufnehmen des biologischen Materials umfassen, wobei die Plattform ferner eine Rahmenstruktur (103) zum mechanischen Unterstützen der Plattformelemente und zum mechanischen Unterstützen eines Gasversorgungssystems umfasst, das in der Lage ist, die Plattformelemente mit Gas zu versorgen, ohne das Gas von einer externen Quelle zu empfangen, und wobei die Rahmenstruktur Gaskanäle (104) umfasst zum Empfangen des Gases von dem Gasversorgungssystem und zum Leiten des Gases zu den Plattformelementen, so dass die Gaszusammensetzung in jeder der Kammern während der Bestrahlung der biologischen Materialien bestimmt wird, **dadurch gekennzeichnet, dass** jedes der Plattformelemente Folgendes umfasst:
- ein erstes Reservoir (223) zur Aufnahme eines Kulturmediums in flüssiger Form,
- einen ersten Flüssigkeitskanal (224) zum Leiten des Kulturmediums in flüssiger Form vom ersten Reservoir zur Kammer des betrachteten Plattformelements und
- einen zweiten Flüssigkeitskanal (225) zum Ableiten des Kulturmediums in flüssiger Form aus der Kammer des betrachteten Plattformelements.

2. System nach Anspruch 1, wobei jedes der Plattformelemente einen Gasraum (221) zum Aufnehmen des Gases und gasdurchlässiges Material (222) zwischen dem Gasraum und der Kammer umfasst.

3. System nach Anspruch 2, wobei der Gasraum (221) von jedem der Plattformelemente die Kammer (220) des betrachteten Plattformelements umgibt und eine Wand zwischen dem Gasraum und der Kammer das gasdurchlässige Material umfasst.

4. System nach Anspruch 2 oder 3, wobei jedes der Plattformelemente Folgendes umfasst:
- einen ersten Gaskanal (227) zum Leiten des Gases von den Gaskanälen zum Gasraum (221) des betrachteten Plattformelements und
- einen zweiten Gaskanal (228) zum Ableiten des Gases aus dem Gasraum des betrachteten Plattformelements.

5. System nach einem der Ansprüche 2 - 4, wobei ein Volumen des Gasraums jedes der Plattformelemente das 0,5- bis 3-fache, vorzugsweise das 0,5- bis 1-fache eines Volumens der Kammer des betrachteten Plattformelements beträgt.

6. System nach einem der Ansprüche 2 - 5, wobei das gasdurchlässige Material (222) Polydimethylsiloxan umfasst.

7. System nach einem der Ansprüche 2 - 6, wobei der Gasraum (221) und die Kammer (220) durch wasserundurchlässige Strukturen voneinander getrennt sind, um zu verhindern, dass Wasser von der Kammer in den Gasraum austritt.

8. System nach einem der Ansprüche 1 - 7, wobei die Gaskanäle (104) angeordnet sind, um das Gas so zu leiten, dass es durch die Plattformelemente fließt.

9. System nach Anspruch 8, wobei die Plattform ferner eine Rückflusssperre (110) umfasst, um zu verhindern, dass Umgebungsluft in einer Richtung entgegengesetzt zu einer Strömungsrichtung des Gases in die Plattformelemente strömt.

10. System nach einem der Ansprüche 1 - 9, wobei die Plattform eine Zellkulturplattform ist und jedes der Plattformelemente ein Kulturelement ist, bei dem die Kammer zur Aufnahme des biologischen Materials eine Kulturkammer zur Aufnahme einer Zellkultur und eines Kulturmediums in flüssiger Form ist.

11. System nach einem der Ansprüche 1 - 10, wobei die Rahmenstruktur eine Vertiefungsplatte (105) umfasst, die Vertiefungen umfasst, die jeweils eines der Plattformelemente aufnehmen können, und einen Deckel (106), der die Gaskanäle umfasst und zum Schließen der Vertiefungen, wenn er auf die Vertiefungsplatte gelegt wird.

12. System nach Anspruch 11, wobei die Vertiefungsplatte (105) einen Hohlraum zum Aufnehmen des Gasversorgungssystems umfasst.

13. System nach einem der Ansprüche 1 - 12, wobei die Rahmenstruktur konfiguriert ist, um ein anderes Gasversorgungssystem mechanisch zu unterstützen, und die Gaskanäle ein Auswahlventilsystem umfassen (107) zum Auswählen, welches der Gasversorgungssysteme aktiviert wird, um das Gas den Plattformelementen zuzuführen.

14. System nach Anspruch 1, wobei jedes der Plattformelemente ein zweites Reservoir (226) umfasst, das mit dem zweiten Flüssigkeitskanal des betrachteten Plattformelements verbunden ist.

15. Verfahren zur Bestrahlung von biologischem Material, **dadurch gekennzeichnet, dass** das Verfahren Folgendes umfasst:
- Halten (301) des biologischen Materials in einer oder mehreren Kammern eines oder mehrerer Plattformelemente eines Systems nach einem der Ansprüche 1 - 14,
- Aufrechterhalten (302) in der einen oder den mehreren Kammern, die das biologische Material enthalten, einer Gaszusammensetzung, die sich von der Umgebungsluft unterscheidet; und
- Aktivieren (303) der Strahlungsquelle des Systems, um mindestens eine der folgenden Strahlungen auf das biologische Material zu lenken: Röntgenstrahlung, ultraviolette Strahlung, Teilchenstrahlung.

## Revendications

1. Système d'exposition d'une matière biologique à un rayonnement, le système comprenant :
- une plate-forme (101) pour retenir la matière biologique, et
- une source de rayonnement (160) pour diriger au moins l'un des rayonnements suivants sur la matière biologique : rayonnement de rayons X, rayonnement ultraviolet, rayonnement de particules,
dans lequel la plate-forme comprend un ou plusieurs éléments de plate-forme (102) comprenant chacun une chambre (220) destinée à contenir la matière biologique, dans lequel la plate-forme comprend une structure de bâti (103) destinée à supporter mécaniquement les éléments de plate-forme et à supporter mécaniquement un système d'alimentation en gaz capable d'alimenter en gaz les éléments de plate-forme sans recevoir de gaz d'une source externe, et la structure de bâti comprend des canaux de gaz (104) destinés à recevoir le gaz provenant du système d'alimentation en gaz et conduire le gaz vers les éléments de plate-forme afin de déterminer une composition de gaz dans chacune des chambres pendant que la matière biologique est exposée au rayonnement, **caractérisé en ce que** chacun des éléments de plate-forme comprend :
- un premier réservoir (223) pour contenir un milieu de culture sous forme liquide,
- une première conduite de liquide (224) pour acheminer le milieu de culture sous forme liquide du premier réservoir à la chambre de l'élément de plate-forme en considération, et
- une seconde conduite de liquide (225) pour acheminer le milieu de culture sous forme liquide hors de la chambre de l'élément de plate-forme en considération.

2. Système selon la revendication 1, dans lequel chacun des éléments de plate-forme comprend un espace pour gaz (221) destiné à contenir le gaz et une matière perméable au gaz (222) entre l'espace pour gaz et la chambre.

3. Système selon la revendication 2, dans lequel l'espace pour gaz (221) de chacun des éléments de plate-forme entoure la chambre (220) de l'élément de plate-forme en considération, et une paroi entre l'espace pour gaz et la chambre comprend la matière perméable au gaz.

4. Système selon la revendication 2 ou 3, dans lequel chacun des éléments de plate-forme comprend :
- une première conduite de gaz (227) pour acheminer le gaz des canaux de gaz à l'espace pour gaz (221) de l'élément de plate-forme en considération, et
- une seconde conduite de gaz (228) pour acheminer le gaz hors de l'espace pour gaz de l'élément de plate-forme en considération.

5. Système selon l'une quelconque des revendications 2 à 4, dans lequel un volume de l'espace pour gaz de chacun des éléments de plate-forme est de 0,5 à 3 fois, de préférence de 0,5 à 1 fois, un volume de la chambre de l'élément de plate-forme en considération.

6. Système selon l'une quelconque des revendications 2 à 5, dans lequel la matière perméable au gaz (222) comprend du polydiméthylsiloxane.

7. Système selon l'une quelconque des revendications 2 à 6, dans lequel l'espace pour gaz (221) et la chambre (220) sont séparés l'un de l'autre avec des structures imperméables à l'eau pour empêcher l'eau de fuir de la chambre vers l'espace pour gaz.

8. Système selon l'une quelconque des revendications 1 à 7, dans lequel les canaux de gaz (104) sont agencés pour amener le gaz à s'écouler à travers les éléments de plate-forme.

9. Système selon la revendication 8, dans lequel la plate-forme comprend en outre une barrière anti-reflux (110) pour empêcher l'air ambiant de s'écouler dans les éléments de plate-forme dans une direction opposée à la direction d'écoulement du gaz.

10. Système selon l'une quelconque des revendications 1 à 9, dans lequel la plate-forme est une plate-forme de culture cellulaire et chacun des éléments de plate-forme est un élément de culture dans lequel la chambre destinée à contenir la matière biologique est une chambre de culture destinée à contenir une culture cellulaire et un milieu de culture sous forme liquide.

11. Système selon l'une quelconque des revendications 1 à 10, dans lequel la structure de bâti comprend une plaque à puits (105) comprenant des puits étant capables de contenir un des éléments de plate-forme, et un couvercle (106) comprenant les canaux de gaz et destiné à fermer les puits lorsque qu'il est placé par-dessus la plaque à puits.

12. Système selon la revendication 11, dans lequel la plaque à puits (105) comprend une cavité destinée à contenir un système d'alimentation en gaz.

13. Système selon l'une quelconque des revendications 1 à 12, dans lequel la structure de bâti est configurée pour mécaniquement supporter un autre système d'alimentation en gaz, et les canaux de gaz comprennent un système de vanne directionnelle (107) destiné à sélectionner lequel des systèmes d'alimentation en gaz est activé pour alimenter le gaz aux éléments de plate-forme.

14. Système selon la revendication 1, dans lequel chacun des éléments de plate-forme comprend un second réservoir (226) raccordé à la seconde conduite de liquide de l'élément de plate-forme en considération.

15. Procédé d'exposition d'une matière biologique à un rayonnement, **caractérisé en ce que** le procédé comprend :
- la retenue (301) de la matière biologique dans une ou plusieurs chambres des un ou plusieurs éléments de plate-forme d'un système selon l'une quelconque des revendications 1 à 14,
- le maintien (302), dans les une ou plusieurs chambres contenant la matière biologique, d'une composition de gaz différente de celle de l'air ambiant, et
- l'activation (303) de la source de rayonnement du système pour diriger au moins l'un des rayonnements suivants sur la matière biologique : un rayonnement de rayons X, un rayonnement ultraviolet, un rayonnement de particules.
